# DEMANDE DE BREVET EUROPEEN

(11) **EP 1 396 494 A1**
(43) Date de publication de la demande: **10.03.2004**
(21) Numéro de dépôt: 03078108.2
(22) Date de dépôt: 24.04.1996
(51) Int. Cl.: C07F 3/02, A61K 33/06, A61P 9/00, A61K 31/19

(54) **(-)hydroxycitrate de magnésium et ses applications**

(30) Priorité: 28.04.1995 FR 9505146
(62) Demande divisionnaire de: 96400864.3
(71) Demandeur: Shrivastava, Ravi, 63118 Cebazat (FR); HYDROXYDASE SOCIETE DES EAUX MINERALES NATURELLES ET DES LABORATOIRES DU BREUIL, 63400 Chamalières (FR)
(72) Inventeur: Shrivastava, Ravi, 63118 Cebazat (FR); Lambropoulos, Patrick, 13007 Marseille (FR)
(74) Mandataire: Santarelli

(57) **Abrégé**

Le (-)hydroxycitrate de magnésium, son utilisation comme médicament et notamment pour la préparation d'un médicament destiné au traitement d'une maladie cardio-vasculaire, une composition pharmaceutique renfermant du (-)hydroxycitrate de magnésium et un excipient pharmaceutiquement acceptable, une association comprenant du (-)hydroxycitrate de magnésium et au moins un métal choisi dans le groupe constitué par les métaux suivants : magnésium, cuivre, cobalt, zinc, nickel, sélénium, silicium, manganèse, lithium et fer, ou du (-)hydroxycitrate de magnésium et au moins une vitamine et application du (-)hydroxycitrate de magnésium ou d'une association ci-dessus à titre de produit diététique, nutritionnel ou cosmétique.

## Description

La présente invention concerne (-)hydroxycitrate de magnésium, et son utilisation comme médicament notamment pour la préparation d'un médicament destiné au traitement d'une maladie cardio-vasculaire.

Le (-)hydroxycitrate (acide (-)hydroxycitrique ou encore acide Garcinia) est le principe actif obtenu de l'extrait de Garcinia indica ou de Garcinia cambogia, arbres du Sud-Est asiatique et plus particulièrement du sud de l'Inde.

Ces deux espèces de plantes renferment directement un stéréo-isomère (-) de l'hydroxycitrate. L'extrait des fruits de ces deux plantes est largement utilisé dans la médecine traditionnelle indienne pour le traitement de diverses maladies.

Un mélange de (-)hydroxycitrate et de chrome est commercialisé aux Etats-Unis en tant que produit diététique pour lutter contre la surcharge pondérale et l'obésité, sous le nom de Citrin@.

Des activités pharmacologiques connues et des procédés de préparation du (-)hydroxycitrate à partir de fruits sont déjà décrits dans la littérature.

Ce produit est en outre commercialisé par différents fabricants tels que la Société FLUKA.

Majeed M. et al dans : "CITRIN® : a revolutionnary herbal approach to weight management" New Editions, Publishing 1675, Rollins Road, Burlingame, CA 94010 Pages 1-69, 1994, décrit un certain nombre d'études qui montrent que le (-)hydroxycitrate réduit le poids corporel par réduction de la synthèse de matière grasse.

De nombreuses personnes dans le monde, par exemple environ 4 millions de personnes en France, souffrent d'hypercholestérolémie. Lorsque les niveaux de cholestérol circulant excèdent les niveaux normaux, en raison de la péroxydation des lipides, le cholestérol et les esters de cholestérol sont capables de s'accumuler dans les cellules des muscles lisses artériels, provoquant leur prolifération, l'accumulation de lipides sur la paroi artérielle (athérome), pouvant engendrer la formation de thrombus, suivie par le blocage d'artères coronaires ou cérébrales.

Les traitements existants (fibrates, inhibiteurs de HMG coenzyme-A réductase, etc ...) s'appliquent à réduire les niveaux de cholestérol circulant mais ne jouent aucun rôle de protection dans les effets pathologiques du cholestérol sur la paroi artérielle, que ce soit en réduisant la prolifération ou en inhibant l'accumulation du cholestérol dans les cellules des muscles lisses artériels.

En outre, en raison de leurs effets secondaires, tels que l'insuffisance hépatique ou rénale, ces médicaments sont utilisés uniquement lorsque les niveaux de cholestérol circulant excèdent largement les limites physiologiques.

C'est pourquoi il serait souhaitable de trouver un produit doué des propriétés suivantes :
- capable de réduire la synthèse de cholestérol,
- capable d'inhiber l'accumulation des lipides dans les cellules des muscles lisses vasculaires,
- capable d'aider à l'élimination des lipides accumulés dans les cellules des muscles lisses vasculaires,
- capable de réduire la prolifération cellulaire due à la réduction des lipides intracellulaires,
- pourvu d'une marge thérapeutique suffisante, et
- dénué d'effets secondaires ou d'effets toxiques à de hautes doses.

Après de longues recherches, la demanderesse a découvert que le (-)hydroxycitrate était capable de potentialiser les effets de minéraux, notamment des métaux impliqués dans les réactions enzymatiques physiologiques, tout particulièrement le zinc, le cuivre, le magnésium, le manganèse, le sélénium et le silicium, et était aussi capable de potentialiser les effets de vitamines, particulièrement les vitamines A, C et E.

Mais, si les produits précités, minéraux et vitamines, ont montré de légers effets de protection vis à vis de l'hypercholestérolémie, ces effets ne sont pas d'un niveau suffisant pour envisager une utilisation thérapeutique. Mais potentialisés par le (-)hydroxycitrate, ils ont des effets tout à fait surprenants.

C'est pourquoi la présente invention a pour objet une association synergique, caractérisée en ce qu'elle comprend du (-)hydroxycitrate et au moins un métal, sous forme ionisée ou non, de préférence sous forme de cation, choisi dans le groupe constitué par les métaux suivants : magnésium, cuivre, cobalt, zinc, nickel, sélénium, silicium, manganèse, lithium et fer, ou du (-)hydroxycitrate et au moins une vitamine, notamment une vitamine antioxydante.

Le (-) hydroxycitrate est de préférence utilisé sous forme d'acide (-) hydroxycitrique.

Des associations synergiques préférées selon l'invention comprennent du (-)hydroxycitrate et en outre au moins deux métaux, notamment deux cations choisis dans le groupe précité et/ou au moins deux vitamines.

Dans des conditions préférentielles, les constituants de l'association synergique selon l'invention sont présents dans les proportions relatives pondérales suivantes : pour une partie de (-)hydroxycitrate, 0,1 à 2 parties d'un sel minéral ou d'un oxyde d'un métal ci-dessus et/ou 0,1 à 1 partie de vitamine(s), sachant que dans un sel minéral ou un oxyde, le métal peut représenter pondéralement de 2 à 50 % en poids, de préférence de 5 à 40 %, particulièrement 10 à 35 %, par rapport en poids du composé considéré.

Parmi les métaux précités, on préfère le sélénium, le magnésium et le silicium, et tout particulièrement ces deux derniers.

C'est pourquoi des associations préférées selon l'invention renferment à titre de métal, du magnésium, du silicium ou du magnésium et du silicium, de préférence sous forme de cations.

Les anions avec lesquels peuvent être combinés les cations ci-dessus sont les anions pharmaceutiquement acceptables comme par exemple ceux dérivés des acides chlorhydrique, bromhydrique, nitrique, sulfurique, phosphorique acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques ou carboxyliques, ou l'acide gluconique.

Les cations peuvent aussi être combinés au (-)hydroxycitrate ; un exemple est, à titre illustratif, le (-)hydroxycitrate de magnésium.

A titre de vitamines, on retient de préférence les vitamines A, C et E et tout particulièrement les vitamines A et E.

Les associations selon la présente invention se présentent de préférence en doses unitaires comprenant chacune au moins 50 mg de (-)hydroxycitrate, de préférence au moins 100 mg de (-)hydroxycitrate, et tout particulièrement environ 250 mg de (-)hydroxycitrate. Avantageusement, une dose unitaire comprend au plus 1 g de (-)hydroxycitrate. En particulier, une forme unitaire se présente sous un volume inférieur à 20 cm³, notamment inférieur à 10 cm³, tout particulièrement inférieur à 1 cm³.

Les associations objet de la présente invention possèdent de très intéressantes propriétés pharmacologiques. Elles sont douées notamment de remarquables propriétés hypolipémiantes, anticholestérol, antiathéromateuses et antioxydantes notamment vis à vis des radicaux libres. Les associations ci-dessus sont en particulier capables de réduire la synthèse de cholestérol, d'inhiber l'accumulation des lipides dans les cellules des muscles lisses vasculaires, d'aider à l'élimination des lipides accumulés dans les cellules des muscles lisses vasculaires, de réduire la prolifération cellulaire due à la réduction des lipides intracellulaires, et en conséquence, diminuer le dépôt de graisses sur l'endothélium vasculaire.

Ces propriétés sont illustrées plus loin dans la partie expérimentale. Elles justifient l'utilisation des associations ci-dessus décrites à titre de médicament.

Les médicaments selon la présente invention trouvent leur emploi par exemple dans le traitement tant curatif que préventif des pathologies induites par l'augmentation du taux de cholestérol telles que la sténose vasculaire, les stries lipidiques, la formation d'athérome, la thrombose et les maladies vasculaires affectant tant la macro- que la microcirculation ainsi que l'hypercholestérolémie.

Ils trouvent aussi leur emploi dans les troubles de la sénescences liés aux effets des oxydants tels que les radicaux libres.

La dose usuelle, variable selon le sujet traité et l'affection en cause, peut être, par exemple, de 100 à 1000 mg par jour par voie orale chez l'homme de (-)hydroxycitrate, pendant au moins 20 jours, associé à 2 à 40 mg de silice colloïdale et à 100 à 1500 mg de gluconate de magnésium.

A titre de médicaments les associations de produits ci-dessus peuvent être incorporées dans des associations pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple les comprimés simples ou dragéifiés, les gélules, les granulés, les caramels, les solutés buvables les suppositoires, les préparations injectables ou pour application locale ; elles sont préparées selon les méthodes usuelles. Le ou les principes actifs peuvent y être incorporés à des excipients habituellement employés dans ces associations pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La présente invention a également pour objet un procédé de préparation d'une association telle que définie ci-dessus caractérisée en ce que l'on mélange l'(-)hydroxycitrate avec au moins un métal sous forme ionisée ou non ou avec au moins une vitamine antioxydante précitée.

La présente invention a également pour objet une association renfermant du (-)hydroxycitrate et au moins un métal sous forme ionisée ou non choisi dans le groupe constitué par les métaux suivants : magnésium, cuivre, cobalt, zinc, nickel, sélénium, silicium, manganèse, lithium et fer, ou du (-)hydroxycitrate et au moins une vitamine, notamment antioxydante, à titre de médicament, c'est à dire une association de composés ci-dessus pour son utilisation dans une méthode de traitement thérapeutique, curatif ou préventif du corps humain ou animal. A titre de traitement thérapeutique, on peut citer notamment les applications et indications connues de ces métaux ou vitamines ; en effet, leurs effets sont potentialisés par le (-)hydroxycitrate.

La présente invention a encore pour objet l'application d'une association renfermant du (-)hydroxycitrate et au moins un métal sous forme ionisée ou non choisi dans le groupe constitué par les métaux suivants : magnésium, cuivre, cobalt, zinc, nickel, sélénium, silicium, manganèse, lithium et fer, ou du (-)hydroxycitrate et une vitamine notamment antioxydante à titre de produit diététique, complément nutritionnel, adjuvant nutritionnel ou encore à titre de produit cosmétique.

L'invention a donc aussi pour objet des compositions diététiques, nutritionnelles ou cosmétiques renfermant une association ci-dessus.

La présente invention a en outre pour objet une association synergique de (-)hydroxycitrate et d'au moins un métal choisi dans le groupe constitué par les métaux suivants : magnésium, cuivre, cobalt, zinc, nickel, sélénium, silicium, manganèse, lithium et fer, ou du (-)hydroxycitrate et/ou d'au moins une vitamine, notamment antioxydante, pour la préparation d'un médicament destiné au traitement d'une maladie cardio-vasculaire, notamment destiné au traitement d'une pathologie induite par le cholestérol.

Dans des conditions préférentielles de réalisation, le médicament destiné au traitement d'une maladie cardio-vasculaire ci-dessus est un médicament à propriétés antioxydantes des lipides.

Dans des conditions tout particulièrement préférées, le médicament ci-dessus est destiné au traitement de l'hypercholestérolémie et des pathologies induites par celle-ci.

Les exemples qui suivent illustrent la présente demande sans toutefois la limiter.

### PARTIE EXPERIMENTALE

### 1) Etude de mortalité de cellules humaines épithéliales et de cellules de muscle lisse artériel du rat.

On a testé les effets antioxydants du (-)hydroxycitrate et de différents minéraux selon le protocole décrit par Michiels et AI : "A new experimental model to study oxygen toxicity", Arch. int. Physiol. Biochem. 94 (5), S13-S18, 1986, en utilisant des hépatocytes de rat, ainsi que des cellules épithéliales humaines et des cellules de muscle lisse artériel de rat.

On a également testé les effets inhibiteurs de ces composés sur la prolifération du muscle lisse artériel et sur l'accumulation de lipides intracellulaires, en utilisant la méthode décrite par Shrivastava et AI dans Meth. Find. Exp. Clin. Pharmacol. 15 (6), pages 345-350, 1993.

Les divers produits ont été respectivement testés aux concentrations suivantes :
- (-)hyddroxycitrate 0,1 mM
- silicium 0,036 mM
- magnésium 0,1 mM
dans le milieu de culture.

Les résultats obtenus sont les suivants :

| REDUCTION EN POURCENTAGES | | | |
|---|---|---|---|
| Produit testé | mort cellulaire | Accumulation des lipides | prolifération des cellules |
| (-)hydroxycitrate | 0 | 16 (±3) | 6 (±4) |
| Magnésium | 6 (±3) | 20 (±9) | 2 (±2) |
| Silicium | 28 (±7) | 32 (±5) | 28 (±6) |
| (-)hydroxycitrate + magnésium | 22 (±4) | 21 (±5) | 10 (±5) |
| (-)hydroxycitrate + silicium | 36 (±5) | 47 (±9) | 39 (±8) |
| (-)hydroxycitrate + magnésium + silicium | 76 (±9) | 89 (±11) | 52 (±10) |

On observe d'une part que le (-)hydroxycitrate seul n'a aucun effet de protection sur la mortalité cellulaire. Le magnésium seul diminue très faiblement cette mortalité, tandis que l'association (-)hydroxycitrate avec le magnésium a un effet plus protecteur. Le silicium seul ou en association avec (-)hydroxycitrate a un effet protecteur aussi prononcé.

Par contre, l'association (-)hydroxycitrate, magnésium et silicium diminue fortement la mortalité cellulaire (76 ± 9 %), prouvant l'effet protecteur de ce mélange contre les dommages oxydatifs.

Le (-)hydroxycitrate est capable de potentialiser fortement les effets des métaux, tout particulièrement l'association de deux métaux sous forme ionisée ou non.

En ce qui concerne l'accumulation des lipides et la prolifération cellulaire, on observe une potentialisation très importante des effets de l'association de deux minéraux, par le (-)hydroxycitrate.

Ces résultats sont tout à fait inattendus.

### 2) Etude sur la mort de cellules humaines épithéliales et de cellules de muscle lisse artériel de rat.

On a testé les effets antioxydants du (-)hydroxycitrate, de différents vitamines et minéraux selon le protocole décrit par Michiels et AI : "A new experimental model to study oxygen toxicity", ARC and Physial Biochem 94 (5, s13-s18 1986) en utilisant des hépatocytes de rat, ainsi que des cellules épithéliales humaines et des cellules de muscle lisse artériel du rat.

On opère comme indiqué dans le test ci-dessus.

Les vitamines ont été testées aux concentrations respectives de

| | |
|---|---|
| (-)hydroxycitrate | 0,1 mM |
| vitamine A (b carotène) | 10 nM |
| vitamine E (a-tocophérol) | 60 nM |

en solution dans le milieu de culture.

Les résultats obtenus sont les suivants :

| REDUCTION EN POURCENTAGES | | | |
|---|---|---|---|
| Produit testé | mort cellulaire | Accumulation des lipides | prolifération des cellules |
| (-)hydroxycitrate | 0 | 16 (±3) | 6 (±4) |
| Vitamine A | 29 (±8) | 23(±6) | 0 |
| Vitamine E | 32 (±6) | 22 (±10) | 0 |
| (-)hydroxycitrate + vitamine A + vitamine E | 56 (±12) | 67 (±14) | 22 (±7) |

Dans ce cas également, on observe une potentialisation des effets des deux vitamines séparées, par le (-)hydroxycitrate.

En particulier, alors que les vitamines testées n'ont aucun effet par elles-mêmes sur la prolifération cellulaire, l'association testée voit ses effets potentialisés, par rapport à l'utilisation de (-)hydroxycitrate seul.

En résumé, ces résultats montrent que le (-)hydroxycitrate, le magnésium, la vitamine A et la vitamine E, seuls, n'ont pas ou peu d'effet vis à vis de la réduction de la mort cellulaire induite par les oxydants, l'accumulation intracellulaire des lipides ou la prolifération de cellules de muscle vasculaire lisse, alors que le silicium a un effet légèrement protecteur sur ces trois paramètres.

Une combinaison de (-)hydroxycitrate avec soit le magnésium, soit le silicium ou deux vitamines a potentialisé de manière très marquée ces effets avec de remarquables effets de protection des cellules contre l'attaque oxydante ainsi que pour réduire l'accumulation de lipides dans les cellules.

L'ensemble des résultats montre que l'exposition des cellules aux composés ((-)hydroxycitrate, un métal ou une vitamine) de manière individuelle a peu ou pas d'effet sur les dommages oxydatifs ou sur l'accumulation lipidique intracellulaire. Mais la combinaison du (-)hydroxycitrate avec des métaux, par exemple des sels minéraux et/ou des vitamines précités a un effet protecteur très prononcé.

Cette découverte importante, par effet de potentialisation entre le (-)hydroxycitrate et les métaux, notamment les sels ou oxydes minéraux, et les vitamines, apporte un bénéfice non négligeable dans le traitement des pathologies cardio-vasculaires liées à l'hypercholestérolémie.

### 3) Toxicité

La toxicité du (-)hydroxycitrate est supérieure à 2 g/kg par voie intrapéritonéale ou à 4 g/kg per os chez le rat. Celle des métaux, notamment sous forme de sels ou d'oxydes minéraux (fréquemment administrés en tant qu'oligo-élément).

### EXEMPLE 1 On a préparé des comprimés répondant à la formule

| | |
|---|---|
| (-)hydroxycitrate (principe actif) | 300 mg |
| Gluconate de magnésium | 360 mg |
| Silice colloïdale | 7,5 mg |
| excipient q.s. pour un comprimé terminé à (détail de l'excipient : lactose, amidon, talc, stéarate de magnésium). | 1 000 mg |

### EXEMPLE 2 On a préparé des comprimés sécables répondant à la formule :

| | |
|---|---|
| (-)hydroxycitrate | 150 mg |
| Vitamine A | 5 mg |
| Vitamine E | 75 mg |
| excipient q.s. pour un comprimé terminé à (détail de l'excipient : lactose, amidon, talc, stéarate de magnésium). | 250 mg |

### EXEMPLE 3 On a préparé des comprimés sécables répondant à la formule :

| | |
|---|---|
| (-)hydroxycitrate | 375 mg |
| gluconate de magnésium | 360 mg |
| silice colloïdale | 7,5 mg |
| sélénium | 40 mg |
| excipient q.s. pour un comprimé sécable terminé à | 1200 mg |

### EXEMPLE 4 On a préparé des ampoules buvables répondant à la formule :

| | |
|---|---|
| (-)hydroxycitrate | 300 mg |
| gluconate de magnésium | 250 mg |
| sélénium | 40 mg |
| excipient q.s. pour une ampoule terminée à (détail de l'excipient : eau distillée) | 10 ml |

### EXEMPLE 5 On a préparé des sachets pour suspension buvable répondant à la formule :

| | |
|---|---|
| (-)hydroxycitrate | 500 mg |
| gluconate de magnésium | 500 mg |
| sélénium | 8 mg |
| excipient q.s. pour un sachet terminé à (détail de l'excipient : lactose) | 5 g |

### EXEMPLE 6 On a préparé des sachets pour suspension buvable répondant à la formule :

| | |
|---|---|
| (-)hydroxycitrate | 250 mg |
| gluconate de magnésium | 250 mg |
| sélénium | 40 mg |
| excipient q.s. pour un sachet terminé à (détail de l'excipient : lactose) | 5 g |

## Revendications

1. Le (-)hydroxycitrate de magnésium.

2. Le (-)hydroxycitrate de magnésium pour son utilisation dans une méthode de traitement thérapeutique, curatif ou préventif, du corps humain ou animal.

3. Utilisation du (-)hydroxycitrate de magnésium pour la préparation d'un médicament destiné au traitement d'une maladie cardio-vasculaire.

4. Une composition pharmaceutique, **caractérisée en ce qu'**elle renferme du (-)hydroxycitrate de magnésium et un excipient pharmaceutiquement acceptable.

5. Une association, **caractérisée en ce qu'**elle comprend du (-)hydroxycitrate de magnésium et au moins un métal sous forme ionisée ou non, choisi dans le groupe constitué par les métaux suivants : magnésium, cuivre, cobalt, zinc, nickel, sélénium, silicium, manganèse, lithium et fer, ou du (-)hydroxycitrate de magnésium et au moins une vitamine.

6. Une association synergique selon la revendication 5, **caractérisée en ce qu'**elle comprend au moins deux métaux choisis dans le groupe constitué par les métaux suivants: magnésium, cuivre, cobalt, zinc, nickel, sélénium, silicium, manganèse, lithium et fer.

7. Une association selon la revendication 5 ou 6, sous forme d'une dose unitaire comprenant au moins 50 mg de (-)hydroxycitrate de magnésium.

8. Une association selon l'une des revendications 5 à 7, **caractérisée en ce qu'**elle renferme pondéralement, pour une partie de (-)hydroxycitrate de magnésium, 0,1 à 2 parties d'un sel minéral ou d'un oxyde d'un métal ou pour une partie de (-)hydroxycitrate de magnésium, 0,1 à 1 partie d'au moins une vitamine.

9. Application du (-)hydroxycitrate de magnésium ou d'une association telle que définie à l'une des revendications 5 à 8, à titre de produit diététique, ou nutritionnel.

10. Application du (-)hydroxycitrate de magnésium ou d'une association telle que définie à l'une des revendications 5 à 8, à titre de produit cosmétique.
